# EUROPEAN PATENT SPECIFICATION

(11) **EP 3 678 692 B1**
(45) Date of publication and mention of the grant of the patent: **20.10.2021**
(21) Application number: 18854165.0
(22) Date of filing: 10.09.2018
(51) Int. Cl.: A61K 39/00, A61K 33/08, A61P 3/10, A61P 37/02, G01N 33/564, C12Q 1/6883

(54) **GENOTYPE STRATIFICATION IN DIABETES TREATMENT AND PREVENTION**
GENOTYPSTRATIFIZIERUNG BEI DER BEHANDLUNG UND VORBEUGUNG VON DIABETES
STRATIFICATION DU GÉNOTYPE DANS LE TRAITEMENT ET LA PRÉVENTION DU DIABÈTE

(30) Priority: 08.09.2017 SE 1751094
(43) Date of publication of application: 15.07.2020
(60) Divisional application: 21195581.0
(73) Proprietor: Diamyd Medical AB, 111 56 Stockholm (SE)
(72) Inventor: ESSEN-MÖLLER, Anders, 115 25 Stockholm (SE)
(74) Representative: Zacco Sweden AB
(86) International application number: PCT/SE2018/050904
(87) International publication number: WO 2019/050465

(56) References cited:
- WO-A1-94/04707
- WO-A2-2015/187087
- JOHNNY LUDVIGSSON ET AL: "GAD65 antigen therapy in recently diagnosed type 1 diabetes mellitus", THE NEW ENGLAND JOURNAL OF MEDICINE, vol. 366, no. 5, 2 February 2012 (2012-02-02), pages 433-442, XP055434070, DOI: 10.1056/NEJMoa1107096
- LUDVIGSSON JOHNNY ET AL: "GAD-treatment of children and adolescents with recent-onset type 1 diabetes preserves residual insulin secretion after 30?months : The GAD-Alum Phase II and III Trials", DIABETES/METABOLISM RESEARCH AND REVIEWS, vol. 30, no. 5, 1 July 2014 (2014-07-01), pages 405-414, XP055796913, GB ISSN: 1520-7552, DOI: 10.1002/dmrr.2503
- JANELLE A NOBLE ET AL: "Genetics of the HLA Region in the Prediction of Type 1 Diabetes", CURRENT DIABETES REPORTS, CURRENT SCIENCE INC, NEW YORK, vol. 11, no. 6, 13 September 2011 (2011-09-13), pages 533-542, XP019971883, ISSN: 1539-0829, DOI: 10.1007/S11892-011-0223-X
- HANNELIUS ULF ET AL: "Efficacy of GAD-alum immunotherapy associated within recently diagnosed type 1 diabetes", DIABETOLOGIA, SPRINGER, BERLIN, DE, vol. 63, no. 10, 5 August 2020 (2020-08-05), pages 2177-2181, XP037238798, ISSN: 0012-186X, DOI: 10.1007/S00125-020-05227-Z [retrieved on 2020-08-05]
- REGNELL SE et al.: "Early prediction of autoimmune (type 1) diabetes", Diabetologia, vol. 6 0 26 May 2017 (2017-05-26), pages 1370-81, XP036267633, DOI: doi:10.1007/s00125-017-4308-1
- KRISCHER JP et al.: "Genetic and Environmental Interactions Modify the Risk of Diabetes-Related Autoimmunity by 6 Years of Age: The TEDDY Study", Diabetes Care, vol. 40, no. 9, 23 June 2017 (2017-06-23), - September 2017 (2017-09), pages 1194-1202, XP055581563, ISSN: 0149-5992, DOI: 10.2337/dc17-0238
- WHERRETT DK et al.: "Antigen-based therapy with glutamic acid decarboxylase (GAD) vaccine in patients with recent-onset type 1 diabetes: a randomised double-blind trial", Lancet, vol. 378, no. 9788, 2011, pages 319-27, XP055434071, DOI: doi:10.1016/S0140-6736(11)60895-7

## Description

### Technical field

The present invention relates to the technical field of medicinal treatment, and in particular to methods for immunotherapeutic treatment of patients based on said patients' genetic profile, as well as compounds and compositions for use in such methods.

### Background art

Type 1 diabetes mellitus (T1DM) is an autoimmune disease characterized by an immune-mediated destruction of the insulin-secreting cells, the beta-cells, of the pancreas. T1DM often has an early onset, already in childhood.

The production of autoantibodies to the beta cells causes a degradation of the beta cells. The degradation process occurs over many years and eventually results in metabolic abnormalities. These abnormalities are first manifested as impaired glucose tolerance and progress to symptomatic hyperglycemia. The antibodies that have been identified in association with the development of T1DM are antibodies to insulin (IAA), GAD65 (GADA including truncated GADA or tGADA), IA-2 (IA-2A) and ZnT8 (ZnT8A).

It has been suggested that administration of alum-formulated glutamic acid decarboxylase can preserve beta-cell function in patients with recent-onset T1DM (Ludvigsson et al., N Engl J Med. 2012 Feb 2;366(5):433-42).

Also insulin has been used as an antigen component in immunotherapy for T1DM (Ali et al, Sci Transl Med. 2017 Aug 9;9(402)).

The 6 year incidence of diabetes-associated autoantibodies in genetically at-risk children has been described by Krischer et al. (Diabetologia. 2015 May;58(5):980-7).

Immunomodulation by a therapeutic medication intended for treatment of diabetes and prevention of autoimmune diabetes is known in the art, *i.a*. from EP1755631 and EP3151853.

### Summary of the invention

The number and type of antibodies are predictive of progression to diabetes.

Patients with different genotypes, have different sets and development of antibodies leading to degradation of the beta cells. This in turn will lead to a difference in progression of the disease, based on which antibody is triggered in the patient.

By identifying the genotype and measuring and quantifying the amount of antibodies against GAD65 and insulin simultaneously, it is possible to customize a vaccine for prevention or treatment of Type 1 Diabetes Mellitus (T1DM).

Thus, in one aspect, the invention relates to a GAD autoantigen for use in a method for treatment or prevention of autoimmune diabetes in an individual, comprising determining the HLA haplotype of the individual; and subjecting the individual to a treatment regimen based on said haplotype, wherein an individual having an HLA DR3-DQ2 haplotype is subjected to treatment with at least a GAD-autoantigen.

In one embodiment, the GAD autoantigen is for use in method comprising determining the HLA haplotype of the individual, determining the specificity of the initially occurring autoantibody related to the autoimmune disease in the individual; and subjecting the individual to a treatment regimen based on said haplotype and specificity of said initially occurring autoantibody.

In one embodiment the autoimmune diabetes, such as Type 1 Diabetes Mellitus, 1,5 diabetes, LADA, LADY, SPIDDM, and PIDM.

In one embodiment the individual having an HLA DR3-DQ2 haplotype, is presenting with GADA first.

In one embodiment an individual having an HLA DR3/4-DQ2/8 genotype is subjected to treatment with both a GAD autoantigen and an insulin autoantigen in the same or separate formulations.

In one embodiment an individual having a HLA-DR3/3-DQ2/2 genotype is subjected to treatment with a GAD antigen.

In one embodiment an individual having a HLA- DR3/4-DQ2/8 genotype is subjected to treatment with a GAD antigen.

In one embodiment an administration of GAD-antigen is one of subcutaneous, intradermal, or intralymphatic.

In one embodiment a GAD antigen is formulated with alum.

In one embodiment an administration of insulin autoantigen is oral, sublingual, intramuscular, intradermal, or intra-lymphatic.

In one embodiment an insulin antigen is formulated with alum or in saline solution.

### Definitions

All terms as used herein are intended to have the meaning given to them by the person of ordinary skill in the art in the context of the disclosure. A few terms are specifically defined below in order to avoid ambiguity.

The term "alum" refers to aluminium hydroxide which is commonly used as an adjuvant in pharmaceutical compositions for use in immunotherapy. "Alum alone" refers to a composition comprising alum but no antigen.

The term "GAD" refers to the protein glutamic acid decarboxylase and includes isoforms of GAD such as GAD38, GAD65 and GAD67, as well as fragments thereof.

The term "insulin", when used for insulin as an autoantigen, shall be construed as including preproinsulin, proinsulin, as well as fragments thereof.

The term T1D includes all types of diabetes where autoantibodies to beta-cell autoantigens are present such as Type 1 Diabetes Mellitus (T1DM), 1,5 diabetes, LADA, LADY, SPIDDM, PIDM and more.

The terms "a" and "an" shall be construed as including both the singular and the plural.

### Detailed description

The etiology of T1DM has yet to be clarified. However the pathogenesis is marked by autoantibodies against insulin (IAA), GAD65 (GADA), IA-2 (IA-2A) or ZnT8 (ZnT8A).

Recent investigations have found that two major groups of children developing islet autoantibodies exist; one that present with IAA as a first autoantibody and this group often include children of low age with HLA genotypes including the DR4-DQ8 haplotype, and the other group often somewhat older children that present with GADA or tGADA first, often of DR3-DQ2-haplotype 2, leading the inventors of the present invention to test the efficacy of GAD-alum versus placebo in these HLA-groups (cf. Example 2).

The HLA (Human Leukocyte Antigen) gene family provides for a group of proteins known as HLA complex. The HLA complex helps the immune system to distinguish self from non-self. Within the HLA complex there are three basic groups, MHC (major histocompatibility complex) class I, MHC class II and MHC class III. The MHC class II genes comprise *HLA-D* genes.

A TEDDY (The Environmental Determinants of Diabetes in the Young) substudy has been performed aimed at identifying genetic and environmental factors that explains the trigger of the first islet autoantibody.

In the study the development of islet autoimmunity and T1DM was studied. Enrolled into the study were children - from newborn infants up to children of age 15 years, with a genetic susceptibility for T1DM.

Participants were submitted to clinical visits every 3 months. At each visit blood was analyzed with regards to GADA, IAA, IA-2A, ZnT8A, DNA, mRNA, infectious agents, HbA1c, PBMC, erythrocytes, storage plasma/serum. Analysis was also performed on urinary samples, nasal swabs, tap water, toenail clippings and salivary cortisol. Stool samples were collected monthly during the first 48 months, and thereafter quarterly.

In addition to the analysis mentioned above, interviews were performed regarding maternal pregnancy diet (FFQof selected foods), smoking; negative life events, parental anxiety, depression, records of infections, medications, immunizations, family history, DNA from First Degree Relatives (FDRs), Physical activity assessment. There was also a re-enrolment of subjects lost.

The aim was to study prenatal factors that may affect the risk for HLA-dependent autoantibody appearance. Non-diabetic mothers of singleton infants (n=6,947) filled out a questionnaire 3-4.5 months after pregnancy. Lower birth weight was defined as the lower 25% of the birth weights of the TEDDY children.

Maternal factors such as smoking, BMI, drinking during the 3rd trimester (>2 drinks/month), and maternal infections (lower respiratory tract infection, skin infection or rash, genital infection). After adjusting for country, T1DM in a FDR, HLA genotypes and gender, neither of these maternal factors were associated with a first islet autoantibody.

Among FDR-children IAA-only as first autoantibody was more common than GADA-only at an early age, but not at a later age

Compared to children with genotype *HLA-DR3*/*4,* children with genotype *HLA-DR3*/*3* showed a reduced risk of manifesting IAA-only as the first islet autoantibody.

Among children with genotype *HLA-DR4*/*4* and *HLA-DR4*/*8,* there was a reduced risk for GADA-only as the first islet autoantibody.

Among girls there was a reduced risk of *IAA-*only as the first islet autoantibody, but not GADA-only as the first islet autoantibody.

Among children born with a lower birth weight, there was a reduced risk of GADA-only as the first islet autoantibody.

The appearance of IAA-only was more common in boys and associated with *HLA-DR4*/*4-DQ8*/*8* and *HLA-DR8*/*4-DQ4*/*8.*

The appearance of GADA-only was not associated with gender, but associated with *HLA-DR3*/*3-DQ2*/*2* and *HLA-DR3*/*4-DQ2*/*8,* and less common in children with low birth weight.

It could be concluded that GADA-only as the first islet autoantibody is triggered primarily in individuals carrying the *HLA-DQ2* haplotype. Maternal factors affecting birth weight may affect the appearance of GADA as the first autoantibody.

Individuals with genotypes *HLA-DR4*/*4-DQ8*/*8* and *HLA-DR8*/*4-DQ4*/*8* generally primarily show presence of IAA as first islet autoantibody.

Individuals with genotypes *HLA-DR3*/*3-DQ2*/*2* and *HLA-DR3*/*4-DQ2*/*8* generally primarily show presence of GADA as first islet antibody

Surprisingly the present inventors could show that administration of a GAD-autoantigen to T1DM patients that had an HLA-haplotype associated with GADA as the first autoantibody, favorably can be used in treatment/prevention regimens for T1DM. The antigen to be used may thus be decided based on the genotype of the patient.

In addition it could be shown that alum alone can delay the decline of stimulated C-peptide in patients carrying the DR4-DQ8, but not the DR3-DQ2 haplotype.

According to the present invention, a GAD autoantigen, and optionally an insulin autoantigen, can be administered by intralymphatic injection, injection directly into a lymph node, intradermal injection, subcutaneous injection, intramuscular injection, intraperitoneal injection, intravenous injection, subcutaneous injection, intranasal, transmucosal or sublingual application; or orally, including administration as tablets, pellets, granules, capsules, lozenges, aqueous or oily solutions, suspensions, emulsions, sprays or as reconstituted dry powdered form with a liquid medium.

In one embodiment of this invention administration of GAD autoantigen, and optionally an insulin autoantigen, is made directly into the lymph nodes or into the lymphatic system to allow resident APCs to present antigen peptides to the immune system. If administration of autoantigens is made directly into a lymph node or into the lymphatic system, the dose will preferably be between 1 and 15 µg per autoantigen, more preferred between 2 and 10 µg per autoantigen or 2 to 5 µg per autoantigen. Formulation in alum is preferred.

According to certain embodiments, the GAD autoantigen, and optionally the insulin autoantigen, is administered intrainguinal, intra-lymph node or intralymphatic. In some embodiments, the volume for intra-inguinal injection of the antigens is between 0.05 and 0.2 ml, more preferred between 0.05 and 0.15 ml.

According to certain embodiments, where the GAD antigen, and optionally the insulin autoantigen, is administered by intralymph-node or intralymphatic injection a preferred dosage is between 1-15ug, more preferred between 2-10, and most preferred between 2-5ug per injection and autoantigen used, such administrations taking place at least 2 times, more preferred at least 3 times and most preferred at least 4 times, at least 14 days apart, more preferably at least 30 days apart.

According to certain embodiments, the GAD antigen or the insulin autoantigen, and at least one IL-10 inducing compound are administered simultaneously. According to certain embodiments, the at least one antigen and the at least one IL-10 inducing compound are administered separately.

The method may further comprise a pre-treatment of the individual to adjust the serum vitamin-D level, and such pre-treatment may comprise administration of vitamin-D and/or vitamin-D analogs, and/or exposure to UVB-radiation, preferably for between 7 to 90 days before administration of the composition comprising the GAD autoantigen to said subject, as described in EP3151853.

### Examples

The examples below aims to further illustrate the invention. The examples shall not be considered as limiting the scope of the invention, which is that defined by the appended claims.

### Example 1: TrialNet Phase II

Wherrett DK et al., (Lancet, 2011 Jul 23;378(9788):319-27) reported from a three arm (A: 3 sc x 20 µg GAD-alum (n=48); B: 2 x 20 µg GAD-alum and 1 of alum (n=49); and C: 3 x alum (n=48), randomized study in subjects diagnosed with T1D within 100 days and aged 3-45 years, that at 1 year, the 2-h AUC of C-peptide, adjusted for age, sex, and baseline C-peptide value, was A 0.412 nmol/L (0.349-0.478) in the GAD-alum group, B 0.382 nmol/L (0.322-0.446) in the GAD-alum plus alum group, and C 0.413 nmol/L (0.351-0.477) in the alum group corresponding to a loss in mean C-peptide at one year of 44%, 42% and 41% of baseline mean for GAD-alum x3, GAD-alum x2, and alum x3 respectively. The authors point out that the levels of C-peptide at baseline and at one year mirrored the findings in the control groups of three other TrialNet studies in subjects treated within 3 months of diagnosis, which also demonstrate that alum alone has no effect on the loss of insulin secretion at 12 months.

Adjusted declines over 12 months are thus 3,7%, 3,5%, and 3,42% monthly. Unadjusted values for up to 24 months are shown in the table below. Here the groups A, B and C showed a decline of and 58,3%, 62,3%, 55,1% over two years at or monthly 2,42%, 2,6%, 2,3%. Alum being marginally best.

**Table 1: Mean AUC C-peptide over 24 months**

| Time | Al*3 | | GAD-Al*2 | | GAD-Al*3 | |
|---|---|---|---|---|---|---|
| | Mean time* (mths) | Mean C-peptide | Mean time* (mths) | Mean C-peptide | Mean time* (mths) | Mean C-peptide |
| Baseline | -0.83 | 0.697 | -0.736 | 0.660 | -0.75 | 0.736 |
| 3 mths | 2.87 | 0.637 | 2.88 | 0.561 | 2.90 | 0.683 |
| 6 mths | 6.12 | 0.537 | 6.03 | 0.530 | 5.98 | 0.545 |
| 9 mths | 9.07 | 0.477 | 9.07 | 0.431 | 9.00 | 0.521 |
| 12 mths | 12.0 | 0.418 | 12.0 | 0.350 | 12.0 | 0.448 |
| 18 mths | 18.0 | 0.365 | 18.1 | 0.284 | 18.0 | 0.334 |
| 24 mths | 24.1 | 0.313 | 24.0 | 0.249 | 23.8 | 0.307 |

However, hypothesizing that GAD as an immunomodulating antigen is efficient in the HLA-groups that usually present with GAD-antibodies first, and that the Insulin-family of antigens is efficient in haplotypes that often present with Insulin antibodies first, the data from the study has now been revisited by the present inventors and it was surprisingly found that GAD-alum was almost twice as effective as alum alone (2,35% versus 4,45% change/month), in the DR3-DQ2 haplotype group that normally present with GAD antibodies first. In addition GAD-alum's efficiency in the groups that include DR4-DQ8 which usually present with IAA first (although DR3/DR4-DQ2/DQ8's may present with either GADA or IAA first) was only at a 40% advantage. And of particular interest was that the adjuvant alum (placebo) alone was more effective than the active drug in the DR4-DQ8 haplotype group, which is reported to include subjects that often present with IAA first.

**Table 2: Change in stimulated C-peptide at 1 year related to haplotype.**

| ***Number of pats* & *DQ*** | ***Arm*** | ***Ab*** | ***Change Stim C-pep AUC at 1 yr (%)*** | ***Change Stim C-pep AUC per month (%)*** |
|---|---|---|---|---|
| 7 with DQ2 | 3 injections 20ug GAD-alum | GADA | -22,03 | -1,84 |
| 7 with DQ2 | 2 injections 20ug GAD-alum plus by one alum alone | GADA | -34,33 | -2,86 |
| *14 with DQ2* | *Average active arms* | *GADA* | -*28,18* | -2,35 |
| 4 with DQ2 | 3 injections alum only | GADA | -53,37 | -4,45 |

| ***Number of pats* & *DQ*** | ***Arm*** | ***Ab*** | ***Change Stim C-pep AUC at 1 yr (%)*** | ***Change Stim C-pep AUC per month (%)*** |
|---|---|---|---|---|
| 19 with DQ8 | 3 injections 20ug GAD-alum | IAA | -42,84 | -3,57 |
| 13 with DQ8 | 2 injections of 20ug GAD-alum plus one alum alone | IAA | -37,21 | -3,10 |
| *32 with DQ8* | *Average active arms* | *IAA* | *-40,55* | *-3,38* |
| 16 with DQ8 | 3 injections alum only | IAA | -22,48 | -1,87 |

### Example2: DiaPrevlt

A randomized double-blind, placebo-controlled study in 50 healthy children with multiple islet autoantibodies but not yet with insulin requiring diabetes (DiaPREV-IT, ClinicalTrials.gov Identifier: NCT01122446), found that preventive treatment with GAD-alum did not affect progression to onset of clinical T1D.

The study was conducted in children aged 4-17.9 years (median age 5.2) with GADA and at least one additional islet autoantibody. Enrollment was completed in 2012 and the follow up period was 5 years. Eligible children, from the Diabetes Prediction in Skåne (DiPiS), The Environmental Determinants of Diabetes in the Young (TEDDY) studies, received two subcutaneous injections of 20 ug GAD-alum(n=25) or placebo (n=25), 30 days apart. Among the exclusion criteria was positivity for HLA DQB1*06:02.

Islet cell autoantibodies predict clinical onset of T1D and a child with more than one islet autoantibody runs a 70 % risk to develop T1D within 10 years. The sample size of 50 children randomized 1:1 to treatment with either GAD-alum (active) or alum alone (placebo), was based upon the assumption that 50 % of untreated children with more than one autoantibody will present with clinical T1D within a 5-year period. Surprisingly however, it was found that, although half of the children had impaired glucose metabolism at baseline, indicating a very high risk of progression to clinical disease, the individuals presenting with clinical T1D during the 5-year follow-up period were only 18 out of 50 (36%) and not 50 % as expected. In light of the reported overall non-significant results, an investigation was performed to evaluate which, if any, subgroups could explain the lower than expected incidence of overt T1D cases being presented.

It was found that 12 of the 18/50 children who progressed to T1D within the 5-year follow-up period (170-1830 days after the first injection of study drug) were girls, having a higher progression rate than boys (p=0.012). Progression to T1D was not affected by being a first degree relative (p=0.925), while children with impaired glucose metabolism at baseline, defined as plasma-glucose 120 min after OGTT ≥7.8 and <11.1 mmol/L, plasma glucose ≥11.1 mmol/L 30, 60 and/or 90 minutes after OGTT and or FPIR <30 in IvGTT, had a higher rate of progression than children with normal glucose tolerance (p=0.013).

Time to clinical diagnosis was not affected by treatment in the full group (p=0.573), or within the stratum groups with 2 or 3-6 autoantibodies (p=0.957 and 0.628 respectively). Moreover, time to diabetes was not significantly affected by treatment within the group of normal and impaired glucose metabolism (p=0.359 and p=0.376, respectively) or by gender (p=0.079 boys, p=0.400 girls, respectively.

Recent investigations have reported that two major groups of children developing islet autoantibodies exist; one that present with IAA as a first autoantibody and this group often include children of low age with HLA genotypes including the DR4-DQ8 haplotype, and the other group often somewhat older children that present with GADA or tGADA first, often of DR3-DQ2-haplotype 2, leading the inventors of the present invention to test the efficacy of GAD-alum versus placebo in these HLA-groups. In this study, including only individuals with high risk to present with clinical T1D, six different HLA-genotypes were represented. Of these the three groups HLA DQ 2/2; DQ 2/X; and DQ X/X included too few subjects (n=2, 2, 1) to be considered in any analysis. The three remaining HLA-groups included DQ 2/8 (n=25) of which 10 subjects received GAD-alum and 15 received placebo; DQ 8/X (n=11) where six subjects received GAD-alum and 5 placebo; and DQ 8/8 (n=9) where five individuals received GAD-alum and 4 placebo.

Surprisingly, it was found that in the 25 subjects in the DQ2/8 group, two of 10 subjects in the GAD-alum group (20%) presented with T1D within the five-year period, compared to eight of the 15 (53%) individuals that received placebo (table 3) indicating that GAD-alum is effective in delaying T1D in high risk individuals with the DQ2/8 haplotype. Similar findings were not seen in the DQ 8/X nor in the DQ 8/8 groups, where placebo came out better than the GAD-alum treated subjects.

**Table 3**

| **25 DQ2/DQ8 positive individuals** | **# individuals presenting with insulin requiring diabetes in the GAD-alum group (n=10)** | **# individuals presenting with insulin requiring diabetes in the Placebo group (n=15)** |
|---|---|---|
| **Time period (year)** | | |
| 1 | 1 | 0 |
| 2 | 0 | 4 |
| 3 | 0 | 3 |
| 4 | 0 | 1 |
| 5 | 1 | 0 |
| 1-5 | 2 (20%) | 8 (53%) |

### Experiment 3: DiagNode-1

As described by Tavira et al., Journal of Diabetes Research, Volume 2018, Article ID 9391845), an open study was conducted in 12 recent onset T1D patients, where 4µg of GAD formulated in alum was injected directly into the inguinal lymphnode at three times, each with a 30 day period in between injections. Consistent with the subject of this invention it was found that treatment of patients carrying the DR3-DQ2 haplotypes resulted in better HbA1c- and stimulated Area Under the Curve C-peptide data, than patients not carrying the DR3-DQ2 haplotypes.

**Table 4**

| **Patient ID-#** | **A1c at baseline (B)** | **A1c % of B at 15 mths** | **Risk group** | **Haplogenotype** | **Stimulated C- peptide at baseline (AUC-B)** | **AUC % of AUC B at 15 mths** |
|---|---|---|---|---|---|---|
| 1 | 52 | 78,8 | Neutral | DR3-DQ2/DR11-DQ7 | 0,364 | 107,56 |
| 2 | 58 | 93,1 | Increased | DR4/4-DQ8/8 | 0,853 | 79,77 |
| 3 | 66 | 78,8 | Increased | DR4/10-DQ8/5.1 | 0,426 | 82,11 |
| 4 | 68 | 60,3 | Decreased | DR11/13-DQ7v/6.3 | 0,744 | 108,40 |
| 5 | 103 | 43,8 | Increased | DR3/4-DQ2/7 | 0,415 | 85,84 |
| 6 | 78 | 56,4 | Increased | DR3/10-DQ2/5.1 | 0,375 | 71,67 |
| 7 | 41 | 90,2 | High | DR3/4-DQ2/8 | | |
| 8 | 37 | 124,3 | Neutral | DR4/10-DQ7/5.1 | 0,518 | 104,83 |
| 9 | 66 | 60,6 | High | DR3/4-DQ2/8 | 0,518 | 76,57 |
| 10 | 54 | 94,4 | Increased | DR4/4-DQ8/8 | 0,356 | 62,11 |
| 11 | 37 | 105,4 | Increased | DR3/13-DQ2/6.4 | 0,853 | 61,73 |
| 12 | 50 | 98 | Increased | DR4/10-DQ8/5.1 | 0,69 | 54,38 |

**Table 5**

| **DR** | **Group Pat ID** | **A1c % of B at 15 mths** | **AUC % of AUCB at 15 mths** |
|---|---|---|---|
| DR3s-pat #11 | 1+5+6+7+9 | 65,964 | 85,41 |
| DR3s | 1+5+6+7+9+11 | 72,53 | 80,67 |
| DR4s | 2+3+8+10+12 | 97,73 | 76,63 |

## Claims

1. A GAD autoantigen for use in a method for treatment or prevention of autoimmune diabetes in an individual, comprising
- Determining the HLA haplotype of the individual; and
- Subjecting the individual to a treatment regimen based on said haplotype,
wherein an individual having an HLA DR3-DQ2 haplotype is subjected to treatment with at least a GAD-autoantigen.

2. The GAD autoantigen for use according to claim 1, comprising
- Determining the HLA haplotype of the individual;
- Determining the specificity of the initially occurring autoantibody related to the autoimmune disease in the individual; and
- Subjecting the individual to a treatment regimen based on said haplotype and specificity of said initially occurring autoantibody.

3. The GAD autoantigen for use according to claim 1 where the autoimmune diabetes is selected from Type 1 Diabetes Mellitus, 1,5 diabetes, LADA, LADY, SPIDDM, and PIDM.

4. The GAD autoantigen for use according to any one of claims 1-3, wherein the individual is further presenting with GADA first.

5. The GAD autoantigen for use according to any one of claims 1-3, wherein an individual having an HLA DR3/4-DQ2/8 genotype is subjected to treatment with both a GAD autoantigen and an insulin autoantigen in the same or separate formulations.

6. The GAD autoantigen for use according to any one of claims 1-3, wherein an individual having a HLA-DR3/3-DQ2/2 genotype is subjected to treatment with a GAD autoantigen.

7. The GAD autoantigen for use according to any one of claims 1-6 wherein an administration of GAD-antigen is one of subcutaneous, intradermal, or intra-lymphatic.

8. The GAD autoantigen for use according to any one of claims 1-7 wherein the GAD antigen is formulated with alum.

9. The GAD autoantigen for use according to any one of claims 1-8, wherein the GAD autoantigen is selected from the group consisting of GAD38, GAD65, and GAD67.

## Patentansprüche

1. GAD-Autoantigen zur Verwendung in einem Verfahren zur Behandlung oder Vorbeugung von Autoimmundiabetes bei einem Individuum, umfassend
- Bestimmen des HLA-Haplotyps des Individuums; und
- Unterwerfen des Individuums einem Behandlungsregime basierend auf dem Haplotyp,
wobei ein Individuum mit einem HLA DR3-DQ2-Haplotyp einem Behandlungsregime mit mindestens einem GAD-Autoantigen unterworfen wird.

2. GAD-Autoantigen zur Verwendung nach Anspruch 1, umfassend
- Bestimmen des HLA-Haplotyps des Individuums;
- Bestimmen der Spezifität des anfänglich vorkommenden Autoantikörpers, der mit der Autoimmunerkrankung bei dem Individuum verbunden ist; und
- Unterwerfen des Individuums einem Behandlungsregime basierend auf dem Haplotyp und der Spezifität des anfänglich vorkommenden Autoantikörpers.

3. GAD-Autoantigen zur Verwendung nach Anspruch 1, wobei der Autoimmundiabetes unter Diabetes Mellitus Typ 1, 1,5-Diabetes, LADA, LADY, SPIDDM und PIDM ausgewählt ist.

4. GAD-Autoantigen zur Verwendung nach einem der Ansprüche 1-3, wobei das Individuum weiter erst mit GADA darstellt.

5. GAD-Autoantigen zur Verwendung nach einem der Ansprüche 1-3, wobei ein Individuum mit einem HLA DR3/4-DQ2/8-Genotyp einer Behandlung mit sowohl einem GAD-Autoantigen und einem Insulinautoantigen in derselben oder separaten Formulierungen unterworfen wird.

6. GAD-Autoantigen zur Verwendung nach einem der Ansprüche 1-3, wobei ein Individuum mit einem HLA-DR3/3-DQ2/2-Genotyp einer Behandlung mit einem GAD-Autoantigen unterworfen wird.

7. GAD-Autoantigen zur Verwendung nach einem der Ansprüche 1-6, wobei eine Verabreichung von GAD-antigen entweder subkutan, intradermal oder intralymphatisch ist.

8. GAD-Autoantigen zur Verwendung nach einem der Ansprüche 1-7, wobei das GAD-Antigen mit Alum formuliert wird.

9. GAD-Autoantigen zur Verwendung nach einem der Ansprüche 1-8, wobei das GAD-Autoantigen aus der Gruppe bestehend aus GAD38, GAD65 und GAD67 ausgewählt ist.

## Revendications

1. Auto-antigène GAD destiné à être utilisé dans une procédé de traitement ou de prévention du diabète auto-immun chez un individu, comprenant
- la détermination de l'haplo-type HLA de l'individu ; et
- la soumission de l'individu à un régime de traitement basé sur ledit haplo-type,
dans lequel un individu ayant un haplo-type HLA DR3-DQ2 est soumis à un traitement avec au moins un auto-antigène GAD.

2. Autoantigène GAD destiné à être utilisé selon la revendication 1, comprenant
- la détermination de l'haplo-type HLA de l'individu ;
- la détermination de la spécificité de l'auto-anticorps initialement produit lié à la maladie auto-immune chez l'individu ; et
- la soumission de l'individu à un régime de traitement basé sur ledit haplo-type et la spécificité dudit auto-anticorps apparaissant initialement.

3. Auto-antigène GAD destiné à être utilisé selon la revendication 1, dans lequel le diabète auto-immun est choisi parmi le diabète sucré de type 1, le diabète 1,5, LADA, LADY, SPIDDM et PIDM.

4. Auto-antigène GAD destiné à être utilisé selon l'une quelconque des revendications 1 à 3, dans lequel l'individu est en outre présenté avec GADA d'abord.

5. Auto-antigène GAD destiné à être utilisé selon l'une quelconque des revendications 1 à 3, dans lequel un individu ayant un génotype HLA-DR3/4-DQ2/8 est soumis à un traitement avec à la fois un auto-antigène GAD et un autoantigène d'insuline dans des formulations identiques ou séparées.

6. Auto-antigène GAD destiné à être utilisé selon l'une quelconque des revendications 1 à 3, dans lequel un individu ayant un génotype HLA-DR3/3-DQ2/2 est soumis à un traitement avec un auto-antigène GAD.

7. Auto-antigène GAD destiné à être utilisé selon l'une quelconque des revendications 1 à 6, dans lequel une administration d'auto-antigène GAD est une parmi l'administration sous-cutané, intradermique ou intra-lymphatique.

8. Auto-antigène GAD destiné à être utilisé selon l'une quelconque des revendications 1 à 7, dans lequel l'antigène GAD est formulé avec de l'alun.

9. Autoantigène GAD destiné à être utilisé selon l'une quelconque des revendications 1 à 8, dans lequel l'auto-antigène GAD est choisi dans le groupe constitué de GAD38, GAD65 et GAD67.
